(19) Europäisches Patentamt
European Patent Office
Office européen des brevets

(11) Veröffentlichungsnummer: **0 230 400**
**A2**

# (12) EUROPÄISCHE PATENTANMELDUNG

(21) Anmeldenummer: **87810028.8**

(22) Anmeldetag: **16.01.87**

(51) Int. Cl.[4]: **A 61 K 31/44**

(30) Priorität: **21.01.86 CH 222/86**
**08.04.86 CH 1362/86**
**17.12.86 CH 5053/86**

(43) Veröffentlichungstag der Anmeldung: **29.07.87**
**Patentblatt 87/31**

(84) Benannte Vertragsstaaten: **AT BE CH DE ES FR GB GR IT LI LU NL SE**

(71) Anmelder: **CIBA-GEIGY AG, Klybeckstrasse 141, CH-4002 Basel (CH)**

(72) Erfinder: **Häusermann, Walter, Dr., En Sendey, CH-1867 Ollon (CH)**
Erfinder: **Maurer, Max, Dr., Mühleweg 16, CH-3280 Murten (CH)**
Erfinder: **Friedel, Thomas, Dr., Lot 32 The High Road, East Blaxland, N.S.W. 2774 (AU)**

## (54) N-3-(5-Trifluormethyl-pyridyl-2-oxy)-phenyl-N'-benzoylharnstoffe zur Bekämpfung von Helminthen an Nutztieren.

(57) Die Verwendung von N-3-(5-Trifluormethylpyridyl-2-oxy)phenyl-N′-benzoylharnstoff-Derivaten der Formel I

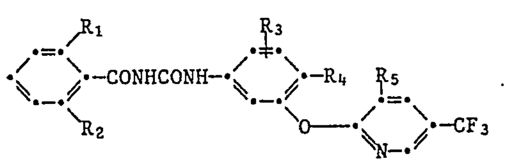

(I)

worin
$R_1$ für Wasserstoff, $C_1$-$C_2$-Alkoxy, $C_1$-$C_2$-Alkylthio, $C_1$-$C_2$-Alkyl oder Halogen steht;
$R_2$ Wasserstoff oder Halogen bedeutet;
$R_3$ für Wasserstoff oder Halogen steht;
$R_4$ Wasserstoff, $C_1$-$C_2$-Alkyl oder Halogen bedeutet; und
$R_5$ für Wasserstoff oder Halogen steht; zur Bekämpfung von Helminthen, insbesondere von Nematoden und Trematoden in Haus- und Nutztieren wird beschrieben.

EP 0 230 400 A2

0230400

CIBA-GEIGY AG
Basel (Schweiz)

5-15720/827/+

N-3-(5-Trifluormethyl-pyridyl-2-oxy)phenyl-N'-benzoylharnstoffe zur Bekämpfung von Helminthen an Nutztieren

Die vorliegende Erfindung betrifft die Verwendung von der nachstehend unter Formel I definierten N-3-(5-Trifluormethyl-pyridyl-2-oxy)phenyl-N'-benzoylharnstoff-Derivate zur Bekämpfung von Helminthen, insbesondere von Nematoden und Trematoden in Haus- und Nutztieren, vor allem in Warmblütern, insbesondere in Säugetieren.

Besagte N-3-(5-Trifluormethyl-pyridyl-2-oxy)phenyl-N'-benzoylharnstoff-Derivate haben die Formel I

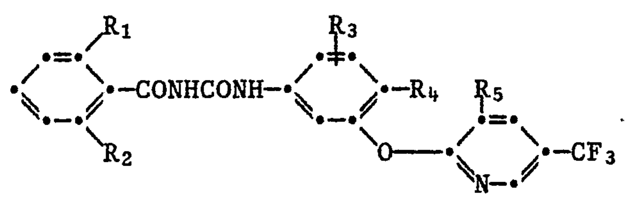

(I)

worin

$R_1$ für Wasserstoff, $C_1$-$C_2$-Alkoxy, $C_1$-$C_2$-Alkylthio, $C_1$-$C_2$-Alkyl oder Halogen steht;

$R_2$ Wasserstoff oder Halogen bedeutet;

$R_3$ für Wasserstoff oder Halogen steht;

$R_4$ Wasserstoff, $C_1$-$C_2$-Alkyl oder Halogen bedeutet; und

$R_5$ für Wasserstoff oder Halogen steht.

Bevorzugt sind Verbindungen der Formel I, worin

$R_1$ für Fluor, Chlor, Methoxy, Methylthio oder Methyl steht;

$R_2$ Wasserstoff, Fluor oder Chlor bedeutet;

$R_3$ Wasserstoff, 2-Fluor oder 2-Chlor bedeutet;

$R_4$ Wasserstoff, Fluor, Chlor, Brom oder Methyl bedeutet; und
$R_5$ für Fluor oder Chlor steht.

Besonders bevorzugt sind Verbindungen der Formel I, worin
$R_1$ für Fluor, Methoxy oder Methylthio steht;
$R_2$ für Fluor steht;
$R_3$ Wasserstoff bedeutet;
$R_4$ Fluor, Chlor oder Brom bedeutet; und
$R_5$ für Chlor steht.

$C_1$-$C_2$-Alkyl steht allein oder als Bestandteil von Alkoxy oder Alkylthio für Methyl oder Ethyl, so dass $C_1$-$C_2$-Alkoxy für Ethoxy oder Methoxy und $C_1$-$C_2$-Alkylthio für Ethylthio oder Methylthio stehen. Halogen bedeutet Fluor, Chlor, Brom, Jod vorzugsweise Fluor, Chlor oder Brom.

Bevorzugte Einzelvertreter der Formel I sind beispielsweise die nachfolgend genannten Substanzen:

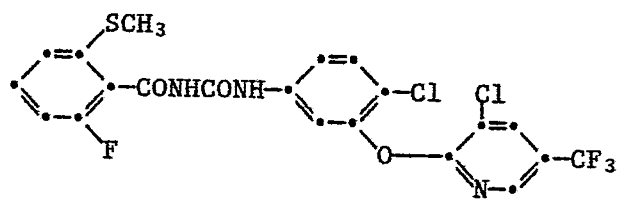

,

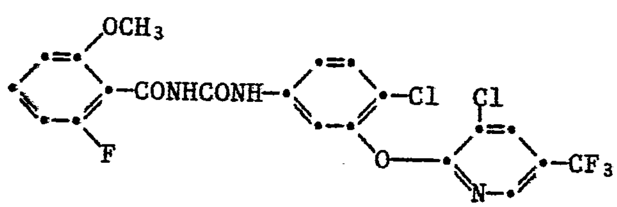

,

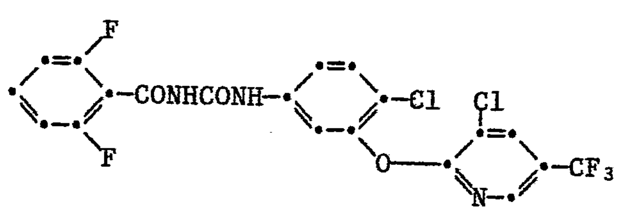

,

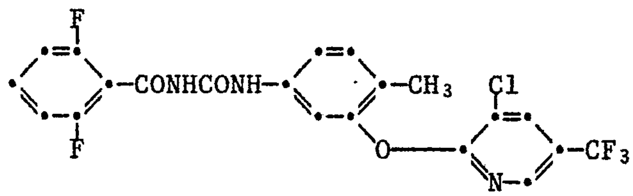

,

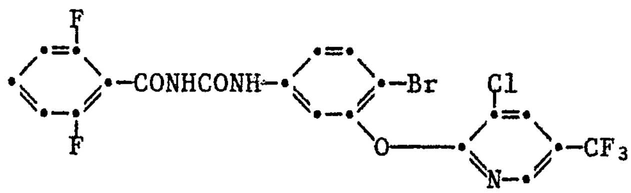

; und

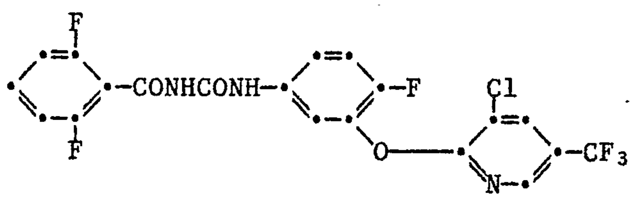

Weitere typische Vertreter von Verbindungen der Formel I sind:

| Verb. Nr. | $R_1$ | $R_2$ | $R_3$ | $R_4$ | $R_5$ |
|---|---|---|---|---|---|
| 1 | F | F | H | $CH_3$ | Cl |
| 2 | F | F | H | H | H |
| 3 | Cl | H | H | H | H |
| 4 | F | F | H | Br | Cl |
| 5 | Cl | Cl | H | Br | Cl |
| 6 | Cl | H | H | Br | Cl |
| 7 | Cl | H | H | $CH_3$ | Cl |
| 8 | H | H | H | $CH_3$ | Cl |
| 9 | F | F | H | Br | H |
| 10 | $CH_3$ | H | H | $CH_3$ | H |
| 11 | Br | H | H | $CH_3$ | Cl |
| 12 | $CH_3$ | H | H | $CH_3$ | Cl |
| 13 | Cl | H | H | $CH_3$ | H |
| 14 | Br | Br | H | $CH_3$ | Cl |
| 15 | F | F | H | $CH_3$ | H |
| 16 | F | F | H | F | Cl |
| 17 | F | F | H | Cl | Cl |
| 18 | F | F | H | F | H |
| 19 | F | F | H | Cl | H |
| 20 | H | H | 2-Cl | H | Cl |
| 21 | H | F | 2-Cl | Cl | Cl |
| 22 | F | Cl | 2-Cl | H | Cl |
| 23 | F | F | H | $C_2H_5$ | Cl |
| 24 | $OCH_3$ | F | H | Cl | Cl |
| 25 | $OCH_3$ | F | H | $CH_3$ | Cl |
| 26 | $OCH_3$ | F | H | $C_2H_5$ | Cl |
| 27 | $OC_2H_5$ | F | H | Cl | Cl |
| 28 | $SCH_3$ | F | H | Cl | F |
| 29 | $SCH_3$ | F | H | $CH_3$ | Cl |
| 30 | $SCH_3$ | F | H | $C_2H_5$ | Cl |
| 31 | $SCH_3$ | F | H | Cl | Cl |
| 32 | $SC_2H_5$ | F | H | Cl | Cl |
| 33 | Cl | Cl | H | Cl | Cl |
| 34 | F | F | H | Cl | Br |
| 35 | F | F | 2-Cl | Cl | Cl |
| 36 | F | F | 3-Cl | Cl | Cl |
| 37 | F | F | 2-F | Cl | Cl |
| 38 | F | F | 2-Br | F | Cl |
| 39 | F | Br | H | Cl | Cl |

In der PCT-Patentanmeldung WO-86/03941 wird das ganze chemische Gebiet der Acylharnstoffe pauschal zur Bekämpfung von Endo- und Ektoparasiten bei Warmblütern beansprucht. Biologische Resultate sind hingegen nur von wenigen Einzelsubstanzen bestimmter Strukturtypen genannt. Hinweise für die hervorragende anthelmintische Wirkung der N-3-(5-Trifluormethyl-pyridyl-2-oxy)phenyl-N'-benzoylharnstoffe der eingangs definierten Formel I fehlen in dieser Publikation. WO-86/03941 tangiert die Verbindungen der Formel I weder strukturell noch wirkungsspezifisch.

Die Verbindungen der Formel I sind als Wirkstoffe zur Bekämpfung von Ektoparasiten aus der Klasse der Insekten sowie der Ordnung Akarina aus der Europäischen Patentanmeldung EP-79311 bekannt oder können wie dort beschrieben hergestellt werden.

So kann man z.B. eine Verbindung der Formel I erhalten, durch Umsetzung

a) einer Verbindung der Formel II

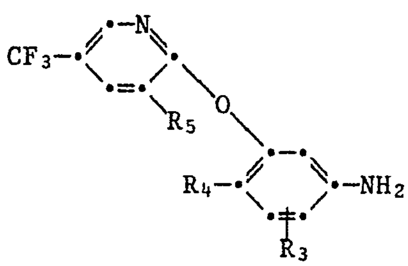

(II)

mit einer Verbindung der Formel III

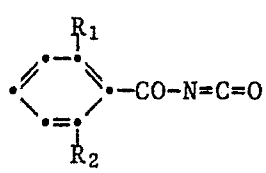

(III)

oder

b) einer Verbindung der Formel IV

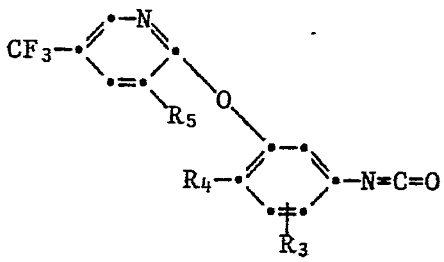

(IV)

gegebenenfalls in Gegenwart einer organischen oder anorganischen Base mit einer Verbindung der Formel V

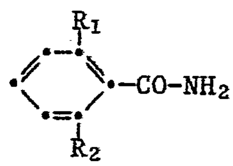

(V),

oder

c) einer Verbindung der Formel II mit einer Verbindung der Formel VI

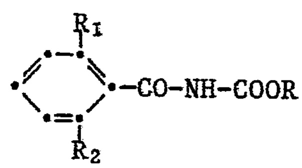

(VI),

In den obigen Formeln II bis VI haben die Reste $R_1$ bis $R_5$ die unter Formel I vorstehend angegebenen Bedeutungen und R bedeutet einen $C_1$-$C_8$-Alkylrest, der gegebenenfalls mit Halogen substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 100°C, vorzugsweise zwischen 15 und 25°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise bei Siedepunkt des verwendeten Lösungsmittels, gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit dem Anilin der Formel II, werden Temperaturen zwischen etwa 60°C und dem Siedepunkt des jeweiligen Reaktionsgemisches bevorzugt, wobei als Lösungsmittel insbesondere aromatische Kohlenmwasserstoffe, wie Toluol, Xylol, Chlorbenzol usw. verwendet werden.

Die Ausgangsstoffe der Formeln III, V und VI sind bekannt und können analog bekannten Verfahren hergestellt werden. Bei den Ausgangsstoffen der Formel II und IV handelt es sich teilweise um neuartige Verbindungen, die nach an sich bkenanten Arbeitsweisen hergestellt werden können (vgl. z.B. die US-Patentschriften Nr. 3.705.170 und 3.711.486).

5-Trifluormethylpyridyl-2-oxy-aniline der Formel II können wie folgt erhalten werden:

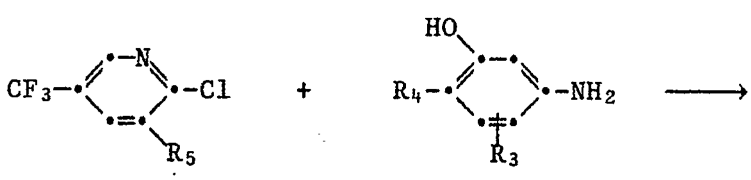 (II).

Diese Umsetzung wird bei einer Temperatur von 20-180°C, vorzugsweise 50-160°C, in Gegenwart eines Säureacceptors, z.B. eines Alkali-oder Erdalkalihydroxids oder -hydrids, vorzugsweise KOH oder NaOH, sowie eines inerten organischen Lösungsmittels, vorzugsweise Dimethylformamid oder Dimethylsulfoxid, durchgeführt. Weiterhin ist ein Anilin der Formel II in Analogie zu dem in J.Org.Chem. 29

(1964), 1, aufgezeigten Verfahren durch Hydrierung der entsprechenden Nitro-Verbindungen herstellbar (vgl. auch die dort zitierte Literatur); auch durch chemische Reduktion (z.B. mittels Sn-(II)-Chlorid/HCl) einer entsprechenden Nitroverbindung sind Aniline der Formel II zugänglich (vgl. Houben Weyl, "Methoden d. org. Chemie" 11-/1, 422):

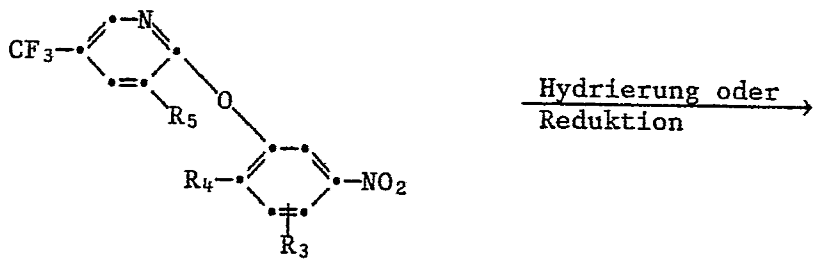

(II).

Zu Benzoylisocyanaten der Formel III kann man unter anderem wie folgt gelangen (vgl. J. Agr. Food Chem. 21, 348 und 993; 1973):

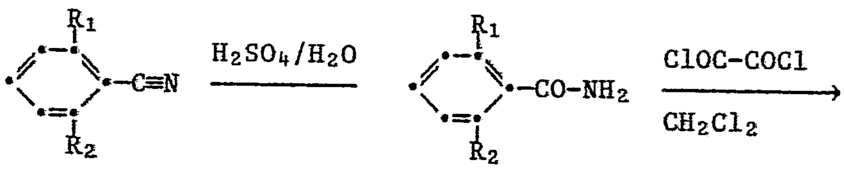

(III).

Ein 3-(5-Trifluormethylpyridyl-2-oxy)-phenylisocyanat der Formel IV lässt sich z.B. durch Phosgenierung eines Anilins der Formel II nach allgemein üblichen Verfahren herstellen. Die weiterhin als Ausgangsstoffe zu verwendenden Benzamide der Formel V sind bekannt (vgl. z.B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure.

Die erfindungsgemäss zu verwendenden Verbindungen der Formel I besitzen überraschenderweise ein sehr günstiges Wirkungsspektrum gegen im Tierorganismus, vor allem in Warmblütern, insbesondere in

Säugetieren, parasitierende Helminthen. Sie sind gegen Nematoden sowie Trematoden mit gutem Erfolg anwendbar. Dabei zeichnen sie sich vor allem dadurch aus, dass sie auch gegen benzimidazol-resistente, insbesondere gegen thiabendazol-resistente Species voll wirksam sind, wobei unter "Thiabendazol" z.B. der Wirkstoff 2-[4-Thiazolyl]-benzimidazol zu verstehen ist. Die anthelmintische Wirkung der Verbindungen der Formel I beruht weniger auf der Abtötung der adulten, parasitären Formen im Körper des Wirtstieres, als vielmehr auf einer entwicklungshemmenden Wirkung auf die Larven und auf die mit dem Kot ausgeschiedenen Eier, so dass der Lebenszyklus des Parasiten wirkungsvoll unterbrochen wird.

Unter den bei Warmblütern vorkommenden Endoparasiten verursachen namentlich die Helminthen grosse Schäden. So können von diesen Parasiten befallene Tiere nicht nur ein verlangsamtes Wachstum, sondern erhebliche physiologische Beeinträchtigungen aufweisen, die sogar zur Mortalität führen können. Daher ist es von grosser Bedeutung, therapeutische Mittel zu entwickeln, die sich zur Bekämpfung von Helminthen und deren Entwicklungsstadien sowie zur Vorbeugung gegen den Befall durch diese Parasiten eignen. Besonders gefährliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen sowie Pferden, Schweinen, Rotwild, Hunden, Katzen und Geflügel auftreten.

Die durch Helminthiasen verursachten Schäden können bei chronischem und vor allem bei epidemischem Auftreten der Wurmerkrankungen in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige vor allem volkswirtschaftlich ins Gewicht fallende Schäden zu vermeiden oder zu mindern.

In der vorliegenden Beschreibung werden unter dem Begriff "Helminthen" insbesondere parasitische Würmer verstanden, die zu den Phyla Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.). Es sind zwar eine Reihe von Stoffen mit anthelmintischer Wirkung bekannt, die für die Bekämpfung der verschiedenen Helminthen species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38, 1425-1426 (1977); Am. J. Vet. Res. 37, 1515-1516 (1976); Am. J. Vet. Res. 38, 807-808 (1977); Am. J. Vet. Res. 38, 1247-1248 (1977)) besitzt zwar ein begrenztes anthelmintisches Wirkungsspektrum bei Wiederkäuern, seine Wirkung gegen Benzimidazol-resistente Nematoden und adulte Leberegel ist jedoch unzureichend, da vor allem die pathologisch wichtigen unreifen Wanderformen der letzteren bei den für das Wirtstier verträglichen Dosierungen nicht angegriffen werden.

Ueberraschenderweise wurde nun festgestellt, dass die Wirkstoffe der Formel I nicht nur - wie bereits erwähnt - eine intensive anthelmintische Wirksamkeit mit breitem Wirkungsspektrum gegen Nematoden, Cestoden und Trematoden, sondern darüber hinaus zusätzlich eine günstige Warmblütertoxizität besitzen.

Die erfindungsgemäss zu verwendenden Wirkstoffe der Formel I sind beispielsweise zur Bekämpfung parasitärer Nematoden der Ordnungen (nach K.I. Skrajabin)

Rhabditida
Ascaridida
Spirurida

Trichocephalida

oder zur Bekämpfung von Cestoden der Ordnungen (nach Wardle & McLeod)

Cyclophyllidae

Pseudophyllidae

oder zur Bekämpfung von Trematoden der Ordnung

Digenea

bei Haus- und Nutztieren wie Rindern, Schafen, Ziegen, Pferden, Schweinen, Katzen, Hunden und Geflügel geeignet. Sie können den Tieren sowohl als Einzeldosis als auch wiederholt verabreicht werden, wobei die einzelnen Gaben je nach Tierart vorzugsweise zwischen 1 und 500 mg pro kg Körpergewicht betragen. Durch eine protrahierte Verabreichung wird in manchen Fällen eine bessere Wirkung erzielt oder man kann mit geringeren Gesamtdosen auskommen.

Die Wirkstoffe der Formel I werden vorzugsweise zusammen mit den in der Formulierungstechnik üblichen Hilfsmitteln eingesetzt und kann daher z.B. zu Emulsionskonzentraten, direkt anwendbaren oder verdünnbaren Lösungen, verdünnten Emulsionen, Spritzpulvern, löslichen Pulvern, Stäubemitteln, Granulaten, auch Verkapselungen in z.B. polymeren Stoffen, Tabletten, Pellets oder Boli in bekannter Weise verarbeitet werden. Die Formulierung, d.h. die einen oder mehrere anthelmintische Wirkstoffe der Formel I und gegebenenfalls feste oder flüssige Zusatzstoffe enthaltenden Mittel, Zubereitungen oder Zusammensetzungen, werden in bekannter Weise hergestellt, z.B. durch inniges Vermischen und/oder Vermahlen des Wirkstoffes mit Streckmitteln, wie z.B. mit Lösungsmitteln, festen Trägerstoffen, und gegebenenfalls oberflächenaktiven Verbindungen (Tensiden).

Zur Bereitung der anthelmintischen Mittel werden z.B. folgende Formulierungshilfsstoffe verwendet:
Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nicht-ionischen

Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Aether und Ester, wie z.B. Aethanol, Aethylenglykol, Aethylenglykolmonomethyl- oder Aethyläther, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxidierte Pflanzenöle wie z.B. epoxidiertes Kokosnussöl oder Sojaöl und Wasser.

Als oberflächenaktive Verbindungen kommen nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können.

Häufig werden sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner können auch entsprechende Phosphate, wie z.B. Salze des Phosphorsäureesters eines p-Nonylphenol-(4-14)-Aethylenoxid-Adduktes oder Phospholipide, als Formulierungshilfsstoffe Anwendung finden.

Als nicht-ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die

genannten Verbindungen enthalten üblicherweise pro Propylenglykol-Einheit 1 bis 5 Aethylenglykoleinheiten.

Als Beispiele nicht-ionischer Tenside seien Nonylphenolpolyäthoxyäthanole, Ricinusölpolyglykoläther,Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" BC Publishing Corp., Ridgewood New Jersey, 1981;
Helmut Stache "Tensid-Taschenbuch" Carl Hanser-Verlag München/Wien 1981.

Als Bindemittel für Tabletten und Boli kommen chemisch abgewandelte, in Wasser oder Alkohol lösliche, polymere Naturstoffe in Frage, wie Stärke-, Cellulose- oder Proteinderivate (z.B. Methylcellulose, Carboxymethylcellulose, Aethylhydroxyäthylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser dem Wirkstoff noch Zusatzstoffe, Vitamine, Antibiotika, Chemotherapeutika, oder andere Pestizide, vornehmlich Bakteriostaika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder der darin enthaltene Wirkstoff der Formel I direkt dem Futter oder den Viehtränken zugesetzt, so enthält das Fertigfutter oder die Fertigtränke den Wirkstoff vorzugsweise in einer Konzentration von etwa 0,0005 bis 0,02 Gewichtsprozent (5-200 ppm).

Die Applikation der Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Boli und Kapseln vorliegen.

Die anthelmintischen Mittel enthalten in der Regel 0,1 bis 99 Gew.%, insbesondere 0,1 bis 95 Gew.-%, eines Wirkstoffes der Formel I und 1 bis 99,9 Gew.-%, insbesondere 5 bis 99,9 Gew.-%, fester oder flüssiger Zusatzstoffe, darunter 0 bis 25 Gew.-%, insbesondere 0,1 bis 25 Gew.-%, an Tensiden.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Herstellungsbeispiele:

Beispiel 1: Herstellung von N-3-(3-Chlor-5-trifluormethyl-pyridyl-2-oxy)-4-chlorphenyl-N'-2,6-difluorbenzoylharnstoff

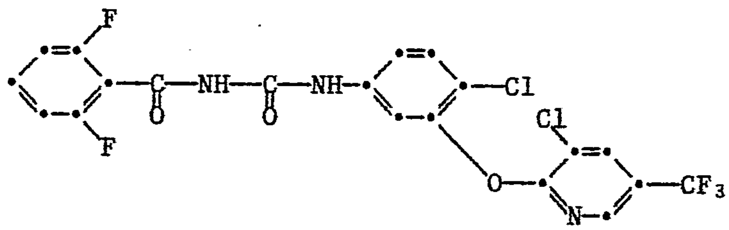

(I)

Zu 6,5 g [3-(3-Chlor-5-trifluormethyl-pyridyl-2-oxy)-4-chloranilin in 50 ml wasserfreiem Toluol lässt man unter Rühren 3,7 g 2,6-Difluorbenzoylisocyanat, gelöst in 25 ml wasserfreiem Toluol, zutropfen. Nach Abklingen der anfänglich exothermen Reaktion, lässt man das Reaktionsgemisch ca. 24 Stunden bei Raumtemperatur stehen, dabei fallen farblose Kristalle aus. Diese werden abfiltriert mit Hexan gewaschen und in Toluol/Hexan umkristallisiert. Man erhält 4,1 g N-3-(3-Chlor-5-trifluormethyl-pyridyl-2-oxy)-4-chlorphenyl-N'-2,6-difluorbenzoylharnstoff. Smp. 217-218°C.

Beispiel 1.1: Herstellung von N-3-(3-Chlor-5-trifluormethyl-pridyl-2-oxy)-4-methylphenyl-N'-2,6-difluorbenzoylharnstoff

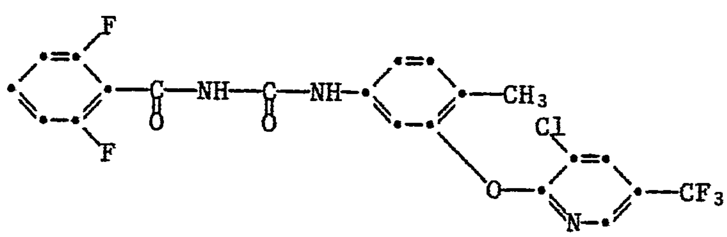

4,5 g 3-(3-Chlor-5-triflourmethyl-pyridyl-2-oxy)-4-methylanilin in 50 ml wasserfreiem Toluol werden mit 2,7 g 2,6-Difluorbenzoylisocyanat in 20 ml wasserfreiem Toluol versetzt. Nachdem die anfänglich exotherme Reaktion abgeklungen ist, wird das Gemisch über Nacht stehengelassen. Nach Filtration und Waschen mit Hexan erhält man in Form weisser Kristalle den N-3-(3-Chlor-5-trifluormethyl-pridyl-2-

oxy)-4-methylphenyl-N'-2,6-difluorbenzoylharnstoff vom Smp. 178-179°C (Verbindung Nr. 1).

Herstellung der Ausgangsverbindung:
Es werden 6,4 g Kaliumhydroxid in 30 ml Dimethylsulfoxid vorgelegt und zu diesem Gemisch eine Lösung von 15,3 g 2-Methyl-5-nitrophenyl gelöst in 30 ml Dimethylsulfoxyd zugetropft. Nach Abklingen der exothermen Reaktion werden tropfenweise 19,9 g eines Gemisches aus 2-Fluor-3-chlor-5-trifluormethylpyridin (40 Gew.%) und 2,3-Dichlor-5-trifluormethylpyridin (60 Gew.%) langsam zugesetzt. Nachdem auch diese exotherme Reaktion abgeklungen ist, wird das Reaktionsgemisch noch während zehn Stunden weiter gerührt. Dann wird das Gemisch auf Eiswasser gegossen und mit Dichlormethan extrahiert. Der Extrakt wird getrocknet und eingedampft. Der Rückstand wird mit einem Gemisch aus Dichlormethan und Hexan (Volumenverhältnis 35:15) über Kieselgel chromatographiert, wobei zu Beginn und am Ende des Chromatographierens reines Dichlormethan als Elutionsmittel verwendet wird. Man erhält das 3-(3-Chlor-5-trifluormethylpyridyl)-2-oxy)-4-methyl-nitrobenzol als weisses kristallines Pulver vom Schmelzpunkt 93-94°C. Nun wird diese erhaltene Verbindung in Dioxan mit Raney-Nickel als Katalysator hydriert. Der Katalysator wird abfiltriert, die als Filtrat aufgefangene Reaktionslösung in Dichlormethan gelöst und über Keiselgel chromatographiert. Man erhält das 3-(3-Chlor-5-trifluormethyl-pyridyl-2-oxy)-4-methylanilin als weisses kristallines Pulver vom Schmelzpunkt 55°C.

<u>Beispiel 2</u>: <u>Formulierungsbeispiele</u>
(% = Gewichtsprozent)

<u>2.1. Emulsions-Konzentrat</u>

| | |
|---|---|
| Wirkstoff Nr. 17 | 10 % |
| Ricinusölthioxilat | 25 % |
| Butanol | 15 % |
| Essigester | 50 % |

Aus diesem Konzentrat können durch Verdünnen mit Wasser Emulsionen jeder gewünschten Konzentration hergestellt werden.

**2.2. Lösungen**

| | a) | b) |
|---|---|---|
| Wirkstoff Nr. 17 | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160-190°C) | - | 94 % |

Die Lösungen sind zur Anwendung in Form kleinster Tropfen geeignet.

**2.3. Granulate**

| | a) | b) |
|---|---|---|
| Wirkstoff Nr. 17 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

**2.4. Stäubemittel**

| | a) | b) | c) | d) |
|---|---|---|---|---|
| Wirkstoff Nr. 17 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff erhält man gebrauchsfertige Stäubemittel.

| 2.5. Spritzpulver | a) | b) | c) |
|---|---|---|---|
| Wirkstoff Nr. 17 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67 % | 27 % | - |

Der oder die Wirkstoffe werden mit den Zusatzstoffen gut vermischt und in einer geeigneten Mühle bis zur Homogenität vermahlen. Man erhält Spritzpulver, die sich mit Wasser zu Suspensionen jeder gewünschten Konzentration verdünnen lassen.

| 2.6. Extruder Granulat | |
|---|---|
| Wirkstoff Nr. 17 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.7. Umhüllungs-Granulat | |
|---|---|
| Wirkstoff Nr. 17 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.8. Suspensions-Konzentrat

| | |
|---|---|
| Wirkstoff Nr. 17 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der oder die fein gemahlenen Wirkstoffe werden mit den Zusatzstoffen innig vermischt. Man erhält so ein Suspensions-Konzentrat, aus welchem durch Verdünnen mit Wasser Suspensionen jeder gewünschten Konzentration hergestellt werden können.

2.9. Pellets bzw. Boli

| | | |
|---|---|---|
| I | Wirkstoff Nr. 17 | 33,0 % |
| | Methylcellulose | 0,80 % |
| | Kieselsäure hochdispers | 0,80 % |
| | Maisstärke | 8,40 % |
| II | Milchzucker krist. | 22,50 % |
| | Maisstärke | 17,00 % |
| | mikrokrist. Cellulose | 16,50 % |
| | Magnesiumstearat | 1,00 % |

I Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und trocknen.

II Alle 4 Hilfsstoffe gut mischen.

III Phasen I und II mischen und zu Pellets oder Boli verpressen.

## 3. Biologische Beispiele

Die anthelmintische Wirksamkeit wird anhand folgender Versuche demonstriert:

### 3.1 Versuche an mit Haemonchus contortus infizierten Schafen

### 3.11 Wirkung auf die Larvenentwicklung im Wirt

An Schafe wird mit Hilfe einer Schlundsonde während 10 Tagen täglich eine bestimmte Menge der zu prüfenden Aktivsubstanz verabreicht. Die Dosierungen variieren zwischen 1 und 10 mg pro kg Lebendgewicht. Die Infektion mit infektiösen H.contortus Larven erfolgt am 2. Tag nach Behandlungsbeginn.

Die Evaluierung der Wirkung erfolgt durch Auszählung der Eier in Kotproben die 21 bis 35 Tage nach der Infektion den Schafen rectal entnommen werden. Die Wirkung zeigt sich darin, dass die Kotproben der mit einer Verbindung der Formel I z.B. Nr. 1, 4, 5, 9, 15 bis 18, 25, 26, 30, 31, 35 und 36 behandelten Schafe, keine Wurmeier enthalten im Gegensatz zu den Kontrolltieren. Das Fehlen von Wurmeiern zeigt, dass die H.contortus sich nicht normal entwickeln können.

### 3.12 Wirkung auf H.contortus Eier an mit adulten H.contortus infizierten Schafen

Massiv mit adulten H.contortus infizierten Schafen wird während 14 Tagen täglich mit Hilfe einer Schlundsonde eine Verbindung der Formel I z.B. Nr. 1, 4, 5, 9, 15 bis 18, 25, 26, 30, 31, 35 und 36 in einer Dosierung von 10 mg pro kg Lebendgewicht verabreicht.

Die Evaluierung der Wirkung erfolgt durch Auszählen der infektiösen Larven in inkubierten Kotproben die zwischen dem 3. und 21. Tag nach Behandlungsbeginn den Schafen rectal entnommen werden. Die Wirkung zeigt sich darin, dass aus zwischen dem 3. und 14. Behandlungstag abgelegten Eiern keine Larven entstehen, wohl aber aus Eiern

unbehandelter Kontrolltiere und aus Eiern, die nach Behandlungsabbruch gelegt werden.

3.13 Wirkung gegen Eier des Leberegels Fasciola hepatica

F.hepatica Eier werden in wässrigem Milieu, Konzentrationen von 7.5, 75 und 750 ppm der Verbindung der Formel I ausgesetzt und bei Raumtemperatur über 15 Tage im Dunkeln aufbewahrt.

Mikroskopische Untersuchung der Eier nach 15 Tagen ergibt, dass sich bei den beiden höheren Konzentrationen keine Miracidien entwickeln und die Eier total verformt sind.

In allen drei Versuchen 3.11, 3.12 und 3.13 zeigen die Verbindungen der Formel I insbesondere die Nr. 1, 4, 5, 9, 15 bis 18, 25, 26, 30, 31, 35 und 36 ausgeprägte entwicklungshemmende Wirkung auf die Eier, Larven bzw. Miracidien (Hemmung 95-100 % im Vergleich zu unbehandeltem Wirt).

Patentansprüche

1. Verwendung von Verbindungen der Formel I

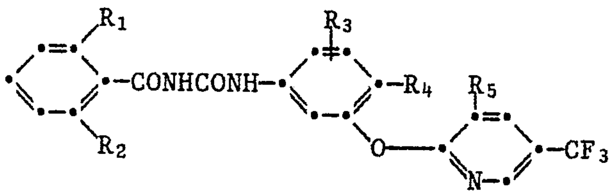

(I)

worin

$R_1$ für Wasserstoff, $C_1$-$C_2$-Alkoxy, $C_1$-$C_2$-Alkylthio, $C_1$-$C_2$-Alkyl oder Halogen steht;

$R_2$ Wasserstoff oder Halogen bedeutet;

$R_3$ für Wasserstoff oder Halogen steht;

$R_4$ Wasserstoff, $C_1$-$C_2$-Alkyl oder Halogen bedeutet; und

$R_5$ für Wasserstoff oder Halogen steht; zur Bekämpfung von Helminthen bei Nutztieren.

2. Verwendung gemäss Anspruch 1, von Verbindungen der Formel I, worin

$R_1$ für Fluor, Chlor, Methoxy, Methylthio oder Methyl steht;

$R_2$ Wasserstoff, Fluor oder Chlor bedeutet;

$R_3$ Wasserstoff, 2-Fluor oder 2-Chlor bedeutet;

$R_4$ Wasserstoff, Fluor, Chlor, Brom oder Methyl bedeutet; und

$R_5$ für Fluor oder Chlor steht.

3. Verwendung gemäss Anspruch 2, von Verbindungen der Formel I, worin

$R_1$ für Fluor, Methoxy oder Methylthio steht;

$R_2$ für Fluor steht;

$R_3$ Wasserstoff bedeutet;

$R_4$ Fluor, Chlor oder Brom bedeutet; und

$R_5$ für Chlor steht.

4. Verwendung gemäss Anspruch 2, einer Verbindung der Formel

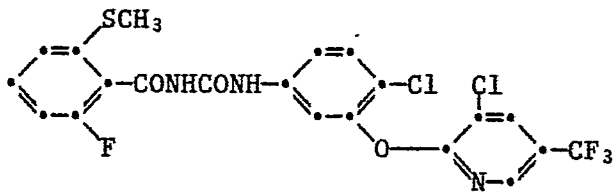

5. Verwendung gemäss Anspruch 2, einer Verbindung der Formel

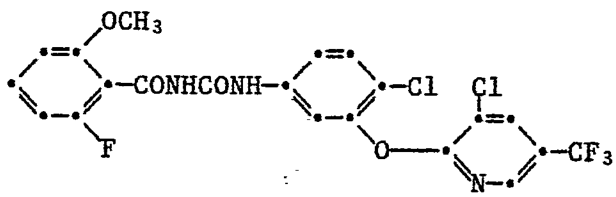

6. Verwendung gemäss Anspruch 2, einer Verbindung der Formel

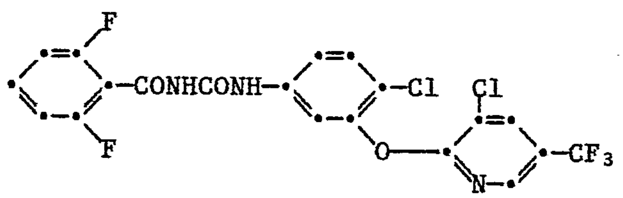

7. Verwendung gemäss Anspruch 2, einer Verbindung der Formel

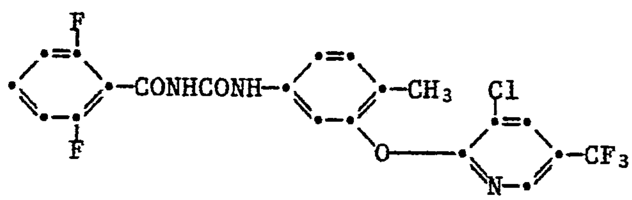

8. Verwendung gemäss Anspruch 2, einer Verbindung der Formel

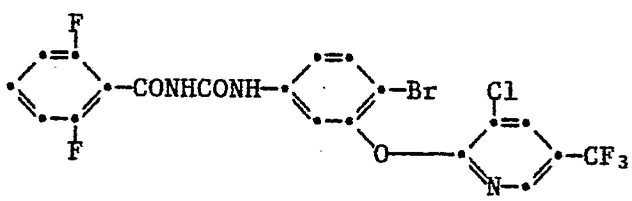

9. Verwendung gemäss Anspruch 2, einer Verbindung der Formel

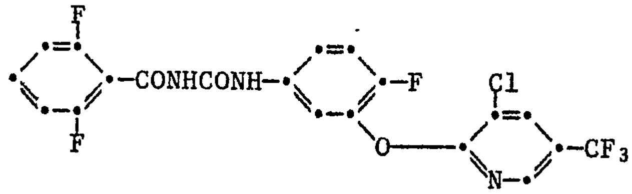

10. Verwendung einer Verbindung der Formel I

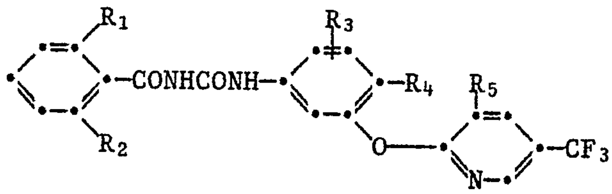 (I)

worin

$R_1$ für Wasserstoff, $C_1$-$C_2$-Alkoxy, $C_1$-$C_2$-Alkylthio, $C_1$-$C_2$-Alkyl oder Halogen steht;

$R_2$ Wasserstoff oder Halogen bedeutet;

$R_3$ für Wasserstoff oder Halogen steht;

$R_4$ Wasserstoff, $C_1$-$C_2$-Alkyl oder Halogen bedeutet; und

$R_5$ für Wasserstoff oder Halogen steht; zur Herstellung eines anthelmintischen Präparates für die Anwendung am Nutztier.

11. Verwendung gemäss Anspruch 10, dadurch gekennzeichnet, dass man als Wirkstoff der Formel I eine Substanz aus den Ansprüchen 2 bis 8 auswählt.

12. Anthelmintisches Tierarzneimittel, dadurch gekennzeichnet, dass es als aktive Komponente mindestens eine Verbindung der Formel I enthält,

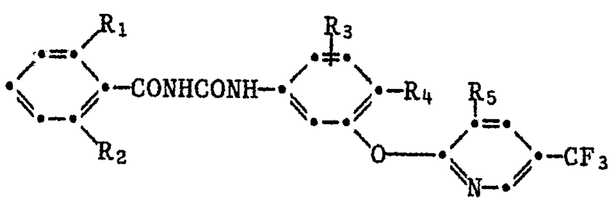 (I)

worin

$R_1$ für Wasserstoff, $C_1$-$C_2$-Alkoxy, $C_1$-$C_2$-Alkylthio, $C_1$-$C_2$-Alkyl oder Halogen steht;

$R_2$ Wasserstoff oder Halogen bedeutet;

$R_3$ für Wasserstoff oder Halogen steht;

$R_4$ Wasserstoff, $C_1$-$C_2$-Alkyl oder Halogen bedeutet; und

$R_5$ für Wasserstoff oder Halogen steht; und Formulierungshilfsstoffe, die für die Applikation in das Tier zweckdienlich sind.

FO 7.5/HL/cw*